# EUROPEAN PATENT APPLICATION

(11) **EP 1 600 161 A2**
(43) Date of publication of application: **30.11.2005**
(21) Application number: 05016274.2
(22) Date of filing: 13.08.1998
(51) Int. Cl.: A61K 31/70

(54) **Processes for preparing 13-deoxyanthracycline derivatives as anticancer of low cardio toxicity**

(30) Priority: 13.08.1997 US 910218
(62) Divisional of application: 98939359.0
(71) Applicant: GEM Pharmaceuticals, Inc., Pelham, AL 35124 (US)
(72) Inventor: Zhang, Xini, Hoover AL 35244 (US); Olson, Richard D., Nampa ID 83561 (US); Walsh, Gerald M., Birmingham AL 35244 (US)
(74) Representative: Pellegri, Alberto

(57) **Abstract**

A high yeld process for preparing 13-deoxyanthracycline derivatives as anticancer of low cardio toxicities, includes the steps of: forming a solution of anthracycline 13-tosylhydrazone; adding a reducing agent to reduce the 13-keto moiety to a methylene moiety; refluxing the solution; cooling the reaction mixture; adding saturated aqueous NaHCO₃; adding a halocarbon solvent to the reaction mixture; filtering the reaction mixture; acidifying the filtrate; and subjecting the filtrate to chromatography to isolate the 13-deoxyanthracycline derivatives.

## Description

### FIELD OF THE INVENTION

The invention relates to methods for preparing 13-deoxyanthracycline derivatives that do not show cardiotoxic side effects.

### BACKGROUND OF THE INVENTION

The anthracyclines have the widest spectrum of activity in human cancers compared to all other cancer chemotherapy. The most well-known anthracycline anticancer drugs are doxorubicin and daunorubicin, which contain a 13-keto group. Of these, doxorubicin, disclosed in U.S. Pat. No. 3,590,028, has a wide spectrum of anticancer utility is one of the most effective drugs in sarcomas and carcinomas, in addition to leukemias, lymphomas, and solid tumors.

The close structural analog, daunorubicin, disclosed in U.S. Patent No. 3,616,242, is not effective in sarcomas and carcinomas and this difference appears to be due to the absence of the 14-OH in daunorubicin. However, daunorubicin, is useful in the treatment of acute leukemias.

However, a cumulative cardiotoxicity limits the utility of these drugs. Along these lines, cardiotoxicity typically limits the duration of doxorubicin treatment to approximately 9 months at usual doses. The total cumulative dose of doxorubicin or daunorubicin typically cannot exceed 550 mg/m² (E.A. Lefrak et al., Cancer, 32:302, 1973). Even at or near the recommended maximum total cumulative dosage (430-650 mg/m²) significant and persistent heart dysfunction occurs in 60% of patients and 14% develop congestive heart failure (A. Dresdale et al., Cancer, 52:51, 1983). Thus, while these drugs are useful to inhibit the growth of cancerous tumors, the patient may die of congestive heart failure because of the severe cardiotoxic side effect of the drugs.

In addition to cardiotoxic effects of the compounds themselves, known processes for preparing anthracycline compounds have relatively low yields, on the order of about 30% (see Smith et al., J. Med. Chem., 21:280-283, 1978).

The following documents relates in general to anticancer compounds including 13-deoxyanthracyclines, their preparation and their anti-tumor activity.
D1: SMITH T H ET AL:'ADRIAMYCIN ANALOGUES. 2. SYNTHESIS OF 13-DEOXYANTHRACYCLINE&EUROPEAN JOURNAL OF MEDICINAL CHEMISTRY, EDITIONS SCIENTIFIOUE ELSEVIER, PARIS, FR, vol. 3, no. 21,1978, pages 280-283, XPOO1 064741 ISSN: 0223-5234
D2: ADAMS N ET AL: 'SYNTHESIS AND ANTITUMOR ACTIVITY OF NOVEL 4-DEMETHOXYANTHRACYCLINES'EUROPEAN JOURNAL OF MEDICINAL CHEMISTRY, EDITIONS SCIENTIFIOUE ELSEVIER, PARIS, FR, vol. 9, no. 33,1990, pages 2375-2379, XP001064742 ISSN: 0223-5234
D3: WO 96 40056 A (SUPRATEK PHARMA INC;KABANOV ALEXANDER V (US)) 19 December 1996 (1996-12-19)
D4: WO 96 40055 A (SUPRATEK PHARMA INC;KABANOV ALEXANDER V (US)) 19 December 1996 (1996-12-19)
D5: NAKAJIMA, SHOHACHI ET AL: 'Synthesis and antitumor activity of 4'-0-acylanthracyclines' J.ANTIBIOT.(1992),45(3),374-9, XPOO1073727
D6: US-A-4 948 880 (HERMENTIN PETER ET AL) 14 August 1990 (1990-08-14)
D7: PATENT ABSTRACTS OF JAPAN vol. 1997, no. 02, 28 February 1997 (1997-02-28) & JP 08 259588 A (MERCIAN CORP), 8 October 1996 (1996-10-08) & DATABASE WPI Derwent Publications Ltd., London, GB;
D8: PATENT ABSTRACTS OF JAPAN vol. 011, no. 300 (C-449), 29 September 1987 (1987-09-29) & JP 62 093298 A (KIRIN BREWERY CO LTD), 28 April 1987 (1987-04-28) & DATABASE WPI Derwent Publications Ltd., London, GB;
D9: US-A-4 710 564 (OTAKE NOBORU ET AL) 1 December 1987 (1987-12-01)
D10: US-A-4 247 545 (CASSINELLI GIUSEPPE ET AL) 27 January 1981 (1981-01-27)
D11: EP-A-0 186 807 (HOECHST AG) 9 July 1986 (1986-07-09)
D12: DATABASE CA [Onlinel CHEMICAL ABSTRACTS SERVICE, COLUMBUS, OHIO, US; SARTORELLI, ALAN C. ET AL: 'Cancer chemotherapeutic agents as inducers of leukemia cell differentiation' retrieved from STN Database accession no. 107:89453 XP002201960 & BRISTOL-MYERS CANCER SYMP. (1987), 8(NEW AVE. DEV. CANCER CHEMOTHER.), 229-44,
D13: DATABASE CA [Online] CHEMICAL ABSTRACTS SERVICE, COLUMBUS, OHIO, US; ADAMS, NIGEL ET AL: 'Synthesis and antitumor activity of novel 4-demethoxyanthracyclines' retrieved from STN Database accession no. 113:152849 XP002201961 & J. MED. CHEM. (1990), 33(9), 2375-9,
D14: DATABASE CA [Online] CHEMICAL ABSTRACTS SERVICE, COLUMBUS, OHIO, US; AJITO, KEIICHI ET AL: 'Improved antitumor effects of 3'-deamino-3'-morpholino derivatives of pirarubicin' retrieved from STN Database accession no. 114:102587 XP002201962 & J. ANTIBIOT. (1990), 43(11), 1464-70.
In particular:
D1 discloses the synthesis of 13-deoxyanthracyclines derivatives (including 13-deoxydoxorubicin and 13-deoxydaunorubicin) and their anti-tumour activity.
D7 and D8 disclose the preparation and use of 13-deoxyanthracycline compounds as anti-tumour agents.
Similarly, the preparation and the use of 13-deoxyanthracycline compounds as anti-tumour agents is disclosed in D6 and D9-D11.

The success of doxorubicin in eliminating tumors and its limitations in clinical use have been the basis for investigators worldwide trying to develop a better doxorubicin. Along these lines, there has been a long felt need for a doxorubicin analog that was not limited by cumulative irreversible cardiotoxicity. Although, over the past 25 years more than 2000 analogues have been synthesized, none have provided a significant improvement over doxorubicin (R.B. Weiss, *The anthracyclines: Will we ever find a better doxorubicin?,* Seminars in Oncology, 19:670-686, 1992).

Extensive research has been performed over the past 25 years to understand the mechanism for anthracycline cardiotoxicity. A popular theory that developed was the free radical theory. According to this theory, cardiotoxicity of anthracyclines results from free radical generation by the quinone moiety of the anthracycline molecule (J. Dorowshow et al., J. Clin. Invest., 68:1053, 1981; D.V. Unverferth et al., Cancer Treat. Rev., 9:149, 1982; J. Goodman et al., Biochem. Biophys. Res. Commun., 77:797, 1977; J.L. Zweier, J. Biol. Chem., 259:6056, 1984).

However, this theory has been disappointing because free radical scavengers and antioxidants have failed to prevent the cumulative cardiac toxicity (D. Propper and E. Maser, *Carbonyl reduction of daunorubicin in rabbit liver and heart,* Pharmacology and Toxicology, 80:240-245, 1997; J.F. VanVleet et al., Am. J. Pathol., 99:13, 1980; D.V. Unverferth et al., Am. J. Cardiol., 56:157, 1985; C. Myers et al., Seminars in Oncology, 10:53, 1983; R.H.M. Julicher et al., J. Pharm. Pharmacol., 38:277, 1986; and E.A. Porta et al., Res. Comm. Chem. Pathol Pharmacol., 41:125, 1983).

In other words, it has been found that inhibition of free radical generation does not eliminate the cardiotoxicity of these anthracyclines (P.S. Mushlin et al., Fed. Proc., 45:809, 1986). Dr. Richard D. Olson and Dr. Phillip S. Mushlin have spent the past 15 years studying the mechanism of anthracycline induced cardiotoxicity and developed the "metabolite theory" which is now expected to become the prevailing theory (R.D. Olson and P.S. Mushlin, *Doxorubicin cardiotoxicity: Analysis* of *prevailing hypotheses,* FASEB Journal, 4:3076-33086, 1990). According to this theory, anthracycline cardiotoxicity is mediated by the 13-OH metabolite of the parent compound.

This research shows that cardiotoxicity of doxorubicin and daunorubicin, as manifested by a reduction in myocardial contractility, is dependent upon the metabolic reduction of the 13-keto moiety to a 13-dihydro metabolite. In test systems where doxorubicin is not metabolized appreciably to the 13-dihydro compound cardiotoxic effects are observed only at very high concentrations (200-400 micrograms/mL) (P.S. Mushlin et al., Fed. Proc., 44:1274, 1985; R.D. Olson et al., Fed. Proc., 45:809, 1986).

If doxorubicin is allowed to remain in the test systems even for short periods of time some metabolic conversion occurs and the 13-dihydro metabolite is formed in sufficient quantity so that cardiotoxicity begins to develop (L. Rossini et al., Arch. Toxicol. suppl., 9:474, 1986; M. Del Tocca et al., Pharmacol. Res. Commun., 17:1073, 1985). Substantial evidence has, thus, accumulated that the cardiotoxicity of drugs such as doxorubicin and daunorubicin results from the potent cardiotoxic effects produced by their 13-dihydro metabolites (P. Mushlin et al., Rational Drug Therapy, 22:1, 1988; S. Kuyper et al., FASEP Journal, 2:A1133, 1988; R. Boucek et al., J. Biol. Chem., 262:15851, 1987; and R. Olson et al., Proc. Natl. Acad. Sci., 85:3585, 1988).

In contrast to the above, the 13-dihydro metabolites, doxorubicinol and daunorubicinol, produce cardiotoxicity in these same test systems at relatively low concentrations (1-2 micrograms/ml, R.D. Olson et al., Proceed. Am. Assoc. Cancer Res., 26:227, 1985; R.D. Olson et al., Proceed. Am. Assoc. Cancer Res., 28:441, 1987).

In view of the above, doxorubicin is converted by intracellular carbonyl reductase to doxorubicinol in which the 13-keto group is reduced to an alcohol as shown below: 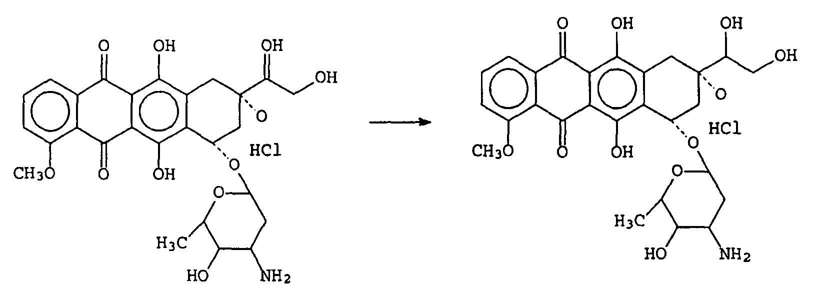 This theory offered an explanation for the time delayed nature of anthracycline cardiotoxicity. The investigations of Olson and Mushlin were reviewed recently and demonstrated a number of points (D. Propper and E. Maser, *Carbonyl reduction of daunorubicin in rabbit liver and heart,* Pharmacology and Toxicology 80: 240-245, 1997).

For example, the investigations bore out a direct relationship between intracardiac C13-alcohol metabolite accumulation and impairment of both contractility and relaxation of the heart muscle. Also, during chronic doxorubicin administration, its 13-alcohol metabolite, doxorubicinol, accumulated selectively in cardiac tissue of rat and rabbit. Additionally, the investigations demonstrate that the in vitro cardiotoxic effect of daunorubicinol is considerably greater than that of the parent drug.

Furthermore, the investigations demonstrate that doxorubicinol was 30 times more potent than doxorubicin in inhibiting cardiac contractility in rabbit papillary muscles. Still further, the investigations demonstrated that the mechanism of cardiac dysfunction was related to ATPase inhibition, since doxorubicinol, but not doxorubicin is a potent inhibitor of Ca²⁺-Mg²⁺-ATPase of sarcoplasmic reticulum, Mg²⁺-ATPase of mitochondria, and Na⁺-K⁺-ATPase activity of sarcolemma. In addition, daunorubicinol, but not daunorubicin, was found in the heart tissue two days after daunorubicin treatment in animal studies.

Recently, the underlying mechanism of anthracycline alcohol metabolite-induced cardiotoxicity was elucidated by Minotti et al., *The secondary alcohol metabolite of doxorubicin irreversibly inactivates aconitase*/*iron regulatory protein-1 in cytosolic fractions from human myocardium,* FASEB Journal, 12:in press, 1998. Minotti et al. demonstrated that doxorubicinol, but not doxorubicin, interferes with iron metabolism and irreversibly inactivates iron regulatory protein-I (IRP-I). As a consequence, iron is not available to iron-requiring enzymes. Inactivation of these enzymes leads to cardiotoxicity.

Consistent with these findings is the fact that the chelator dexrazoxane is useful in reducing doxorubicin cardiotoxicity (G. Weiss et al., *Modulation of transferrin receptor expression by dexrazoxane (ICRF-187) via activation of iron regulatory protein,* Biochemical Pharmacology, 53:1419-1424, 1997). Dexrazoxane appears to stimulate the activity of IRP-I which counteracts the effect of doxorubicinol.

Olson and Mushlin conceived of the idea that an analog of a 13-keto anthracycline that could not form the alcohol metabolite would not be cardiotoxic. The most attractive possibility was to reduce the 13-keto group to a methylene group. There are no enzymes known that will metabolize this group to an alcohol. Consistent with this idea were results obtained with the anthracycline aclarubicin.

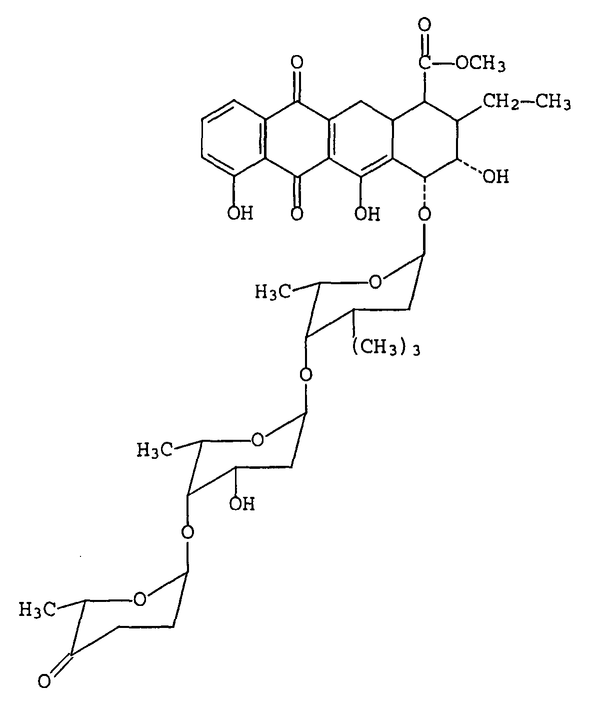

Aclarubicin has numerous modifications compared to doxorubicin, including the absence of a 14-OH group. This drug was not effective against sarcomas or carcinomas, consistent with its lack of a 14-OH group. However, it was effective against acute leukemias. Aclarubicin also has no 13-keto moiety, but, rather, includes a 13-methylene group.

This drug is used commercially in France and Japan. Aclarubicin is apparently devoid of irreversible cumulative cardiotoxicity. Patients have received up to 3,000 mg/m² with no evidence of cardiac dysfunction or cardiomyopathy (D.C. Case et al., *Phase II study of aclarubicin in acute* *myeloblastic leukemia,* American Journal of Clinical Oncology, 10:523-526, 1987). Experts in the field did not understand the lack of cardiotoxicity of aclarubicin and incorrectly assumed that this was due to its distribution and pharmacokinetics. However, Olson and Mushlin believed it confirmed the importance of to the lack of a 13-keto moiety in cardiotoxicity.

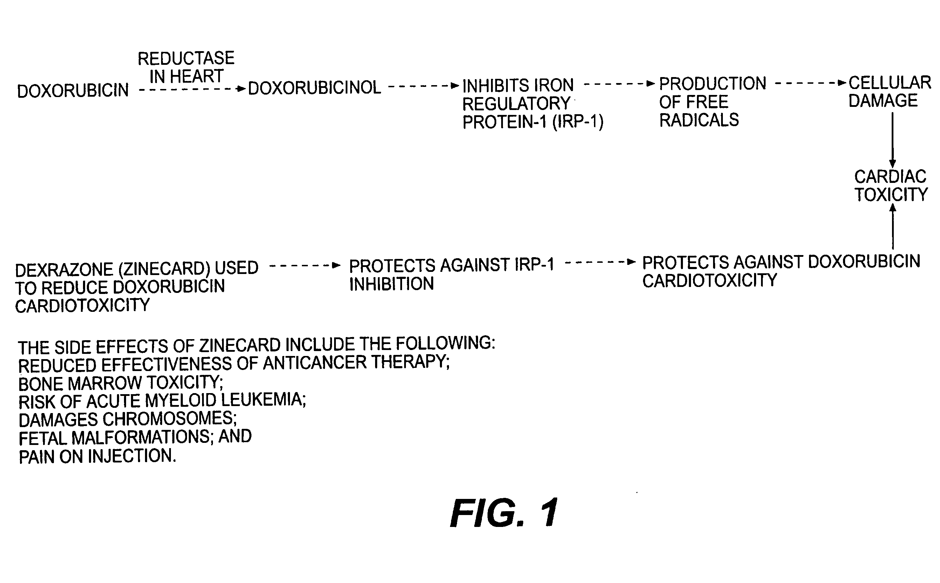

Fig. 1 provides a flowchart that illustrates a route through which cardiotoxicity results.

### SUMMARY OF THE INVENTION

One object of the present invention is to provide improved processes for preparing 13-deoxyanthracycline derivatives.

A further object of the present invention is to provide precursors to certain 13-deoxyanthracycline derivatives and methods for preparing the precursors.

In accordance with these objects, the present invention provide methods for preparing 13-deoxyanthracycline derivatives having the formula 1: 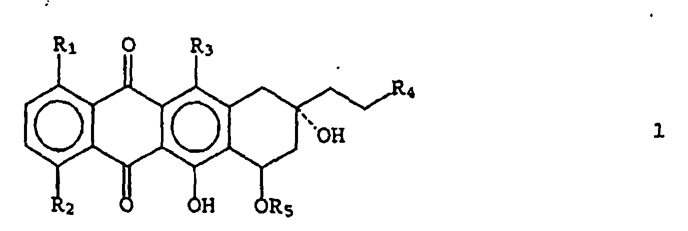 wherein R₁ is H or OH; R₂ is H, OH, or OMe; R₃ is H or OH; R₄ is H or OH; and R₅ is a carbohydrate or substituted carbohydrate. The present invention also provides pharmaceutically acceptable salts of the compounds of formula 1. The pharmaceutically acceptable salts include salts derived from pharmaceutically acceptable inorganic and organic acids and bases. Examples of suitable acids include hydrochloric, hydrobromic, sulfuric, nitric, perchloric, fumaric, maleic, phosphoric, glycollic, lactic, salicyclic, succinic, toluene-p-sulphonic, tartaric, acetic, citric, methanesulphonic, formic, benzoic, malonic, naphthalene-2-sulfonic, trifluoroacetic, and benzenesulphonic acids. Salts derived from appropriate bases include alkali, such as sodium and ammonia.

The present invention also provide an improved anticancer for treating mammalia hosts in need of an anticancer treatment. An effective anticancer amount of at least one compound of the formula 1 below is administered to the host in an effective anticancer amount. 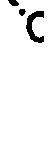 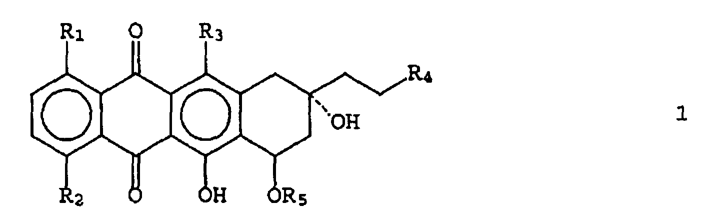 wherein R₁ is H or OH; R₂ is H, OH, or OMe; R₃ is H or OH; R₄ is H or OH; and R₅ is a carbohydrate or substituted carbohydrate.

A process for preparing the 13-deoxyanthracycline derivatives includes forming an acidic solution of anthracycline 13-tosylhydrazone with cyanoborohydride as a reducing agent. The solution is gently refluxed. The reaction mixture is cooled. Saturated aqueous NaHCO₃ is added to the solution, followed by a halocarbon solvent. The mixture is filtered. The filtrate is acidified. The filtrate is subjected to preparative chromatography to isolate the 13-deoxyanthracycline derivatives.

The present invention aims to solve the above-described deficiencies of known processes for preparing 13-deoxyanthracycline derivatives.

Generally, anthracyclines of the formula I 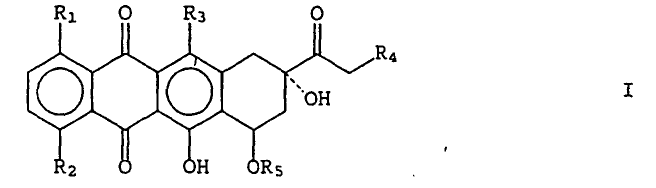 wherein R₁, R₂, R₃, R₄, and R₅ are as defined above. Anthracyclines are readily converted to 13-tosylhydrazones according to known methods. Anthracycline 13-tosylhydrazones are reduced to 13-deoxyanthracycline derivatives with sodium cyanoborohydride under acidic conditions. The products are purified by preparative chromatography without extraction steps. The processes have been found to have a yield of from about 70% to about 80%.

A process for the preparation of 13-deoxyanthracycline derivatives includes forming an acidic solution of anthracycline 13-tosylhydrazone with cyanoborohydride. The solution is gently refluxed.' The reaction mixture is cooled. Saturated aqueous NaHCO₃ is added to the solution, followed by a halocarbon solvent. The mixture is filtered. The filtrate is acidified. The filtrate is subjected to preparative chromatography to isolate the 13-deoxyanthracycline derivatives.

A process for the preparation of 13-deoxyanthracycline derivatives includes forming a solution by dissolving about 1 g of doxorubicin 13-tosylhydrazone hydrochloride and about 2.4 g of p-toluenesulfonic acid in about 50 mL of anhydrous methanol. About 0.8g of sodium cyanoborohydride is added to the solution. The solution is heated to a temperature of from about 68°C to about 72°C. The solution is gently refluxed for about one hour under a nitrogen atmosphere. The reaction mixture is concentrated to about 20 mL. The reaction mixture is cooled in a freezer to a temperature of from about 0°C to about 4°C. About 2 mL of saturated aqueous sodium bicarbonate is added to the reaction mixture. About 200 mL of chloroform is added to the reaction mixture. Anhydrous sodium sulfate is added to the reaction mixture. Salts are filtered out. The filtrate is acidified with hydrogen chloride in diethyl ether. The solution is run through a silica gel column. The column is further washed with chloroform/methanol until the eluate is colorless. A fraction containing the product is eluted with methanol. The methanol eluate is evaporated. Residue resulting from the evaporation is dissolved in 30% acetonitrile in ammonium formate buffer. The product is isolated by preparative HPLC using a phenyl column. The product is separated from other impurities using an acetonitrile/ammonium formate gradient. The HPLC purified fraction is then lyophilized to produce about 600 mg of 13-deoxydoxorubicin hydrochloride.

### BRIEF DESCRIPTION OF THE DRAWINGS

Fig. 1 represents a flowchart illustrating pathways resulting in cardiotoxicity;
Fig. 2 represents a graph illustrating the concentration of doxorubicinol in right atrial and ventricular preparations incubated in 175 µM of an embodiment of a compound according to the present invention or doxorubicin with respect to time;
Fig. 3 represents a graph illustrating [³H]-thymidine uptake in HL-60 cells of an embodiment of a compound according to the present invention or doxorubicin, showing growth inhibition of the cells;
Fig. 4 represents a graph illustrating [³H]-thymidine uptake in P388 cells of an embodiment of a compound according to the present invention or doxorubicin, showing growth inhibition of the cells;
Fig. 5 represents a graph illustrating [³H]-thymidine uptake in MCF7 cells of an embodiment of a compound according to the present invention or doxorubicin, showing growth inhibition of the cells;
Fig. 6 represents a graph illustrating [³H]-thymidine uptake in MDA-MB-231 cells of an embodiment of a compound according to the present invention or doxorubicin, showing growth inhibition of the cells;
Fig. 7 represents a graph that illustrates the effect of Compound B of the present invention and daunorubicin on contractile function; and
Figs. 8a-c represent, respectively, photomicrographs at 200 magnification illustrating histopathology of left ventricular tissue obtained from rabbits treated for 20-23 weeks with an embodiment of a compound according to the present invention, treated with doxorubicin, or a control sample, illustrating myocyte vacuolization and myofibrillar loss in the doxorubicin sample, not seen in the Compound A of the present invention or control samples.

### DESCRIPTION OF BEST AND VARIOUS MODES FOR CARRYING OUT THE INVENTION

The present invention makes use of the fact that the 13-deoxy forms of doxorubicin, daunorubicin, or other similar anthracyclines will not be metabolically converted to cardiotoxic 13-dihydro forms, thus providing a means for administering compounds of the present invention in noncardiotoxic amounts without limitation of total cumulative dosage.

The present invention relates to a method of preparing an improved doxorubicin having the formula A: 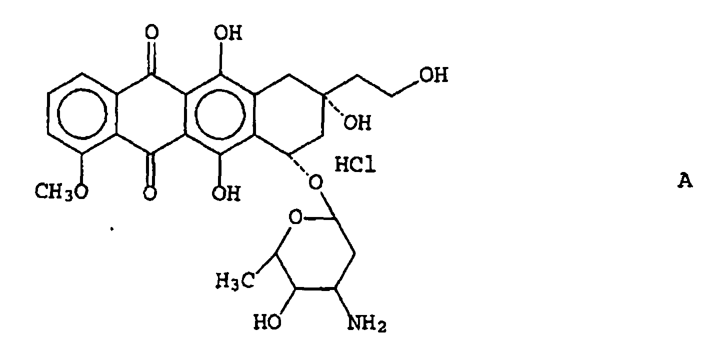 hereinafter referred to as Compound A.

The improved Compound A was synthesized from doxorubicin by reducing the 13-keto moiety to a methylene group. In vitro experiments demonstrated that the improved Compound A was not metabolized by heart tissue to doxorubicinol under the same conditions that doxorubicin was converted to this metabolite.

The in vitro experiments described below studied the biotransformation of the improved doxorubicin of the present invention. The purpose of the study was to determine whether Compound A according to the present invention is metabolized to the C-13 hydroxy metabolite in isolated rabbit cardiac muscle preparations. Doxorubicin, doxorubicinol and Compound A were assayed in right atrial and right ventricular free wall strips obtained from New Zealand white rabbits using fluorescence HPLC techniques. Thin atrial and ventricular strips were incubated in muscle baths (30°C) containing oxygenated Krebs-bicarbonate buffer. Doxorubicin (175 µM) or Compound A (175 µM) was added to the baths and atrial and ventricular strips were removed at 30 minute intervals for 210 minutes. Each strip was briefly washed in normal saline, blotted dry, cut in half and weighed. The tissues were stored in vials at -70°C for determination of doxorubicin, doxorubicinol and Compound A tissue concentrations. Tissue concentrations of doxorubicin, doxorubicinol and Compound A were determined from standard curves in three separate experiments and expressed as mean ±SEM. A time dependent increase in atrial and ventricular concentration of Compound A and doxorubicin was observed. After 210 minutes of incubation, atrial tissue concentrations (ng/mg wet weight) of Compound A and doxorubicin were not significantly different (Compound A: 743±89; doxorubicin: 617±35). Concentrations of Compound A in ventricle were significantly higher than ventricular concentrations of doxorubicin after 210 minutes of incubation (Compound A: 626±31; doxorubicin: 407±30; P<0.05). However, only doxorubicin was metabolized to the C-13 hydroxy metabolite, doxorubicinol. No metabolism of Compound A was detected. These experiments indicate that Compound A does not form the C-13 hydroxy metabolite in isolated cardiac preparations.

The purpose of the study was to determine whether Compound A is metabolized to the C-13 hydroxy metabolite in isolated rabbit cardiac muscle preparations.

The tests were conducted using Compound A according to the present invention and doxorubicin.

Assays were conducted utilizing whole rabbit ventricle, right and left atrial tissue, right ventricular free wall, Krebs-bicarbonate buffer (pH 7.4), Normal (0.9%) saline, (NH₄)₂SO₄, isopropyl alcohol, chloroform, daunorubicin, doxorubicin, doxorubicinol, and methanol.

The test protocol included: thin strips (80 to 100 mg each) of right ventricular free wall and atria from NZW rabbits were incubated in isolated muscle baths (30°C) containing oxygenated Krebs-bicarbonate buffer (pH 7.4) of the following composition: 127 mM NaCl, 2.5 mM CaCl₂, 2.3 mM KCl, 25 mM NaHCO₃, 1.3 mM KH₂PO₄, 0.6 mM MgSO₄ and 5.6 mM glucose as previously reported (P.S. Mushlin et al., Br. J. Pharmacol., 110:975-982, 1993).

According to the doxorubicinol synthesis, doxorubicinol was synthesized by the method of Takanashi and Bachur (S. Takanashi and N.R. Bachur, Drug Metab. Disp., 4:17-87, 1976) with slight modification (P.S. Mushlin et al., Br. J. Pharmacol., 110:975-982, 1993).

According to the statistical analysis of the results of the experiments, tissue concentrations (ng/mg wet weight) of doxorubicin, doxorubicinol and Compound A were determined in three different experiments and expressed as mean ±SEM. A two-factor analysis (ANOVA) was employed to analyze the effects of treatment at various time intervals using Prizm (GraphPad) program.

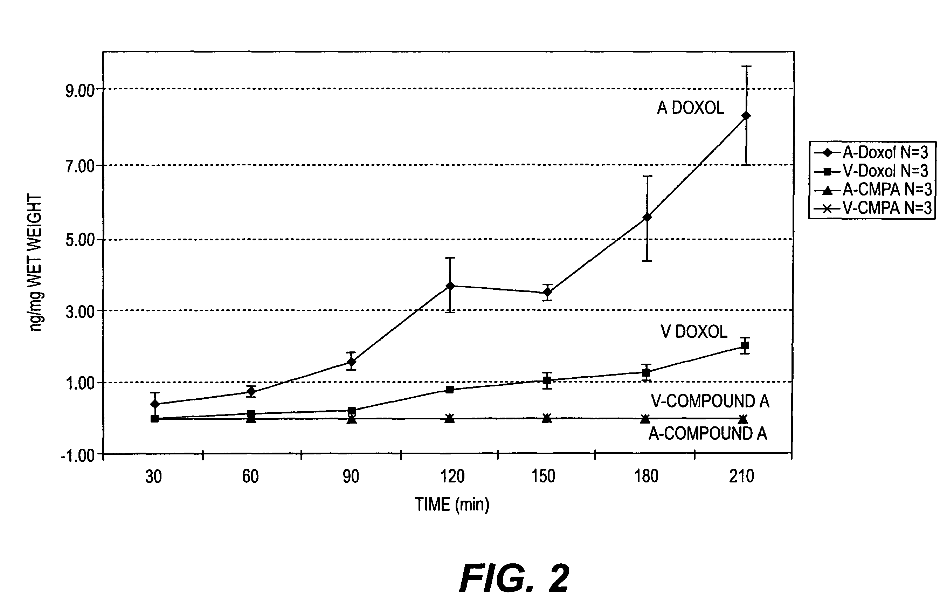

Observations and examination results include observation of a time dependent increase in concentration of Compound A and doxorubicin in both rabbit atrial (Table 1) and right ventricular free wall (Table 2) tissues (Figure 1). Concentrations of Compound A in both atria and ventricles were equal to or higher than atrial and ventricular concentrations of doxorubicin. However, only doxorubicin was metabolized to the C-13 hydroxy metabolite, doxorubicinol and a time dependent accumulation of doxorubicinol was observed in both rabbit atrial (Table 1) and ventricular (Table 2) tissue (Figure 2). No metabolism of Compound A was detected (Figure 2).

**Table 1**

| Concentrations (ng/mg wet weight) of test compounds and doxorubicinol in rabbit atrial tissue. | | | | |
|---|---|---|---|---|
| TIME (Min) | Compound A | Compound A C13 METABOLITE | DOXORUBICIN | DOXORUBICINOL |
| 30 | 242±46 | ND | 179±14 | 0.41±0.32 |
| 60 | 412±65 | ND | 256±22 | 0.73±0.10 |
| 90 | 453±32 | ND | 361±50 | 1.56±0.24 |
| 120 | 518±47 | ND | 434±51 | 3.71±0.77 |
| 150 | 550±34 | ND | 542±29 | 3.53±0.23 |
| 180 | 624±20 | ND | 584±41 | 5.58±1.15 |
| 30 | 242±46 | ND | 179±14 | 0.41±0.32 |
| 60 | 412±65 | ND | 256±22 | 0.73±0.10 |
| 90 | 453±32 | ND | 361±50 | 1.56±0.24 |
| 120 | 518±47 | ND | 434±51 | 3.71±0.77 |
| 210 | 743±89 | ND | 617±35 | 8.31±1.30 |
| ND indicates no detectable concentration of Compound A C-13 hydroxy metabolite. Tissue concentrations obtained from three separate experiments are expressed as mean ±SEM. ND and mean values were obtained from three separate experiments. | | | | |

**Table 2**

| Concentrations (ng/mg wet weight) of test compounds and doxorubicinol in rabbit right ventricular free wall tissue. | | | | |
|---|---|---|---|---|
| TIME (Min) | Compound A | Compound A C13 METABOLITE | DOXORUBICIN | DOXORUBICINOL |
| 30 | 183±9 | ND | 129±19 | 0.03±0.03 |
| 60 | 287±28 | ND | 251±34 | 0.12±0.06 |
| 90 | 412±87 | ND | 221±21 | 0.22±0.03 |
| 120 | 425±23 | ND | 331±27 | 0.80±0.08 |
| 150 | 443±19¹ | ND | 349±26 | 1.04±0.21 |
| 180 | 489±13 | ND | 377±47 | 1.27±0.21 |
| 210 | 626±31¹ | ND | 407±30 | 2.00±0.20 |

| | | | | |
|---|---|---|---|---|
| ¹P>0.05, Compound A vs. doxorubicin. ND indicates no detectable concentration of Compound A C-13 hydroxy metabolite. Tissue concentrations obtained from three separate experiments are expressed as mean ±SEM. ND and mean values were obtained from three separate experiments. | | | | |

Discussion and conclusions from the experiments include that the results of the experiments indicate that Compound A does not form the C-13·hydroxy metabolite in isolated rabbit cardiac preparations. However, doxorubicin, a structurally related compound was metabolized to doxorubicinol, a C-13 hydroxymetabolite. In addition, the results of the experiments indicate that the metabolism of doxorubicin to doxorubicinol appears to be greater in atrial than ventricular tissue.

Fig. 2 illustrates the concentration of doxorubicinol in right atrial (A) and ventricular (V) preparations incubated in 175µM Compound A according to the present invention or doxorubicin over time. As can be seen in Fig. 2, the compound according to the present invention is present in much less concentrations as compared to the known doxorubicin.

Other in vitro studies showed that Compound A according to the present invention was as effective as doxorubicin in inhibiting the growth of human cancer cells.

The experiments demonstrating the effectiveness of the compound provided with the method of the present invention at inhibiting the growth of cancer cells compared the effects of Compound A according to the present invention and doxorubicin on cell proliferation in vitro.

According to the experiments demonstrating the effectiveness of the compound of the present invention, the anti-proliferative effect of Compound A was compared with doxorubicin in cultured cell lines derived from human and murine leukemia (HL60 and P388) and human breast cancer (MCF7 and MDA-MB 231). Inhibition of cancer cell proliferation was studied by measuring cellular incorporation of [³H] thymidine. The anti-proliferative effect of Compound A was compared with doxorubicin under the same culture conditions. Concentration producing 50% of maximum inhibition (IC₅₀) was obtained from curve fitting analysis. Mean IC₅₀ values (in nM) and 95% confidence intervals are shown below. Means were determined from 3 or 4 separate assays repeated in triplicate.

| Cell line | Compound A | Doxorubicin | Potency Ratio |
|---|---|---|---|
| HL60 | 127(108-149) | 58(47-70) | 2.2 |
| P388 | 1980(1830-2140) | 269(242-299) | 7.4 |
| MCF7 | 72(68-77) | 17(17-18) | 4.2 |
| MDA-MB231 | 182(81-408) | 43(34-53) | 4.2 |

Both Compound A and doxorubicin completely abolished [³H] thymidine incorporation into cells in each of the four cell lines studied. These studies demonstrate that both Compound A and doxorubicin are potent inhibitors of proliferation of cancer cells in vitro, although as shown by the ratio of IC₅₀ values (potency ratio), doxorubicin was somewhat more potent than Compound A to inhibit [³H] thymidine incorporation in all four cell lines (P<0.05).

The purpose of the study included determining the potency of Compound A to inhibit cell growth (proliferation) in a number of cultured malignant cell lines derived from human and murine leukemia (HL60 and P388) and human breast cancer (MCF7 and MDA-MB 231), using a well established thymidine incorporation protocol (E. Severison and E.L. Larsson, Lymphocyte responses to polyclonal B and T cell activators, in D.M. Weir (Ed.), Cellular Immunology, Vol 2, Fourth edition, Blackwell Scientific Publications, p. 631, 1986), to calculate an effective concentration producing 50% of the maximum response (IC₅₀) for the test compound in each of four cell lines, and to compare that value with the value obtained for doxorubicin.

Dilutions of test compounds were made up in cell-specific media over the following ranges:

| Cell line | Compound A | Doxorubicin |
|---|---|---|
| HL60 | 10-300nM | 1.25-100nM |
| P388 | 0.1-5µM | 0.05-1.5µM |
| MCF7 | 0.025-2µM | 0.025-2µM |
| MDA-MB231 | 10-300nM | 1.8-240nM |

The dilutions were added to all wells in triplicate as 50µl aliquots and cells grown in the presence of the test compounds for 24 hours.

Statistical analysis included unpaired t-test where appropriate. Level of significance was chosen as P<0.05.

Observations and examination results were as follows. In the four cancer cell lines tested, Compound A and doxorubicin produced concentration dependent inhibition of (³H) thymidine uptake. Concentrations producing 50% of the maximum response (IC₅₀) values were obtained from curve fitting analysis. IC₅₀ values (in nM) are shown below and reflect the mean (with 95% confidence limits in parentheses) of 3-4 assays repeated in triplicate.

| Cell line | Compound A | Doxorubicin |
|---|---|---|
| HL60 | 127(108-149) | 58(47-70) |
| P388 | 1980(1830-2140) | 269(242-299) |
| MCF7 | 72(68-77) | 17(17-18) |
| MDA-MB231 | 182(81-408) | 43(34-53) |

The results indicate that Compound A is less potent than doxorubicin to inhibit cellular proliferation in all four cell lines in vitro. However, both compounds are equally efficacious.

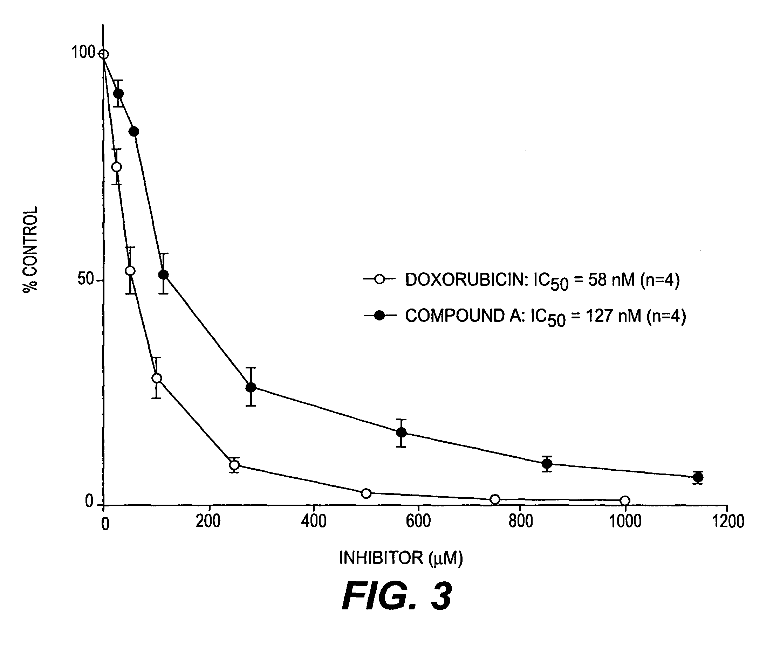

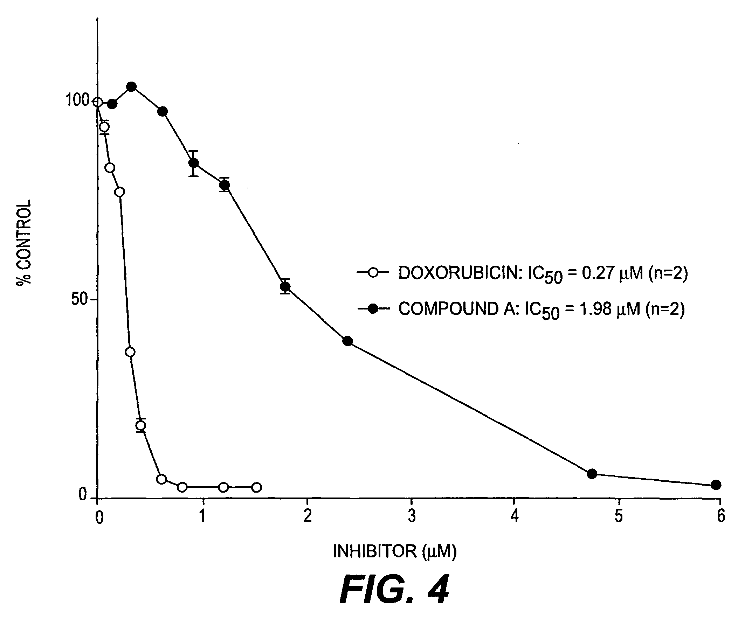

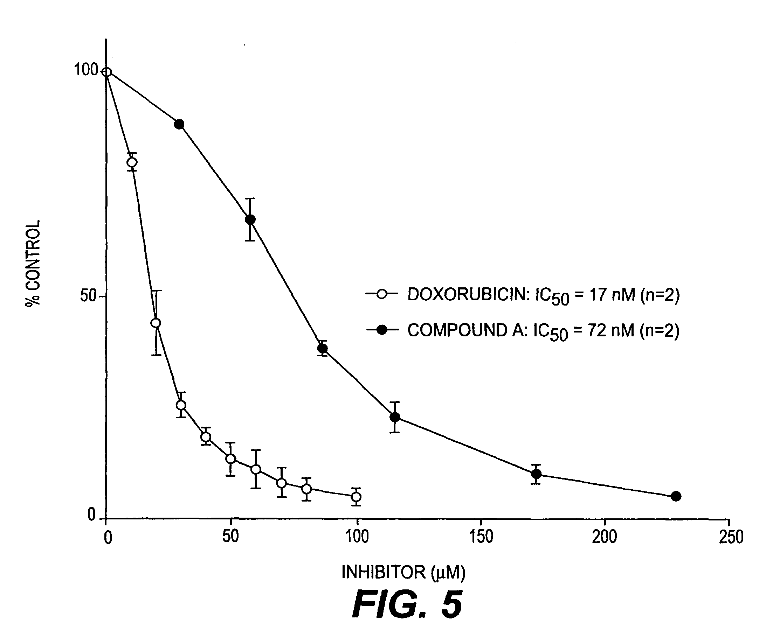

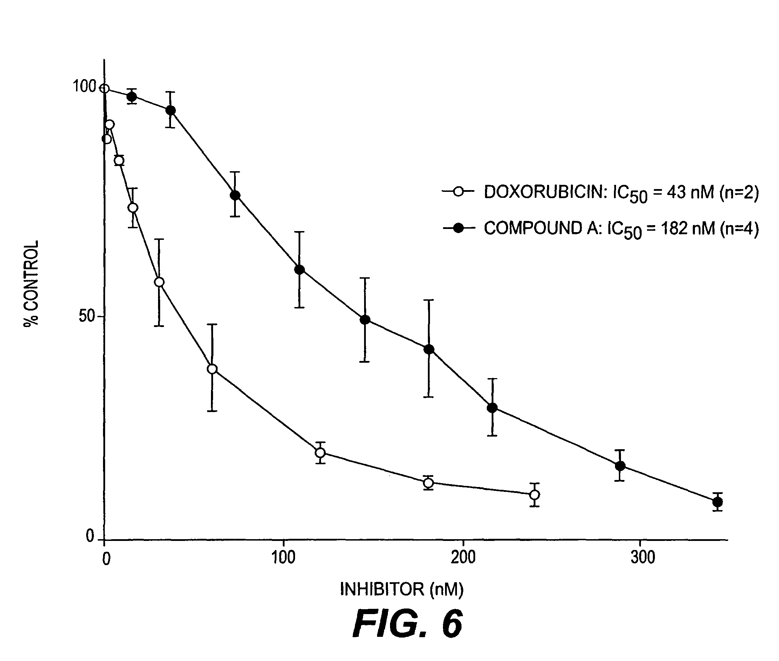

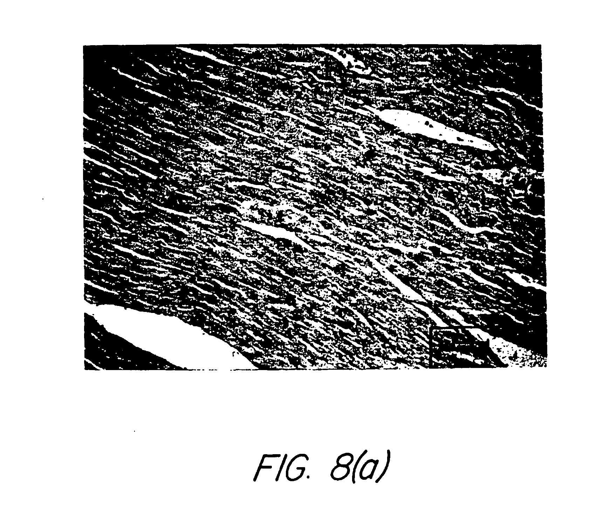

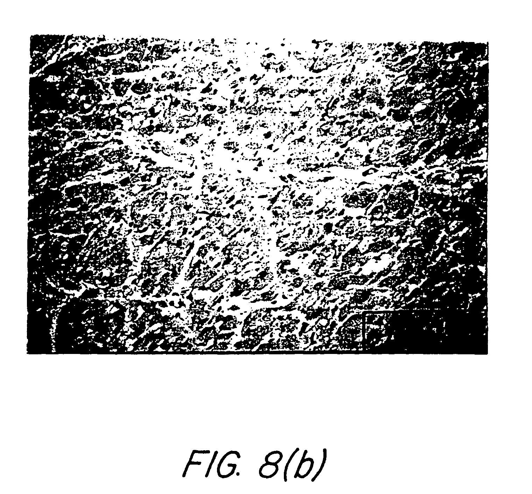

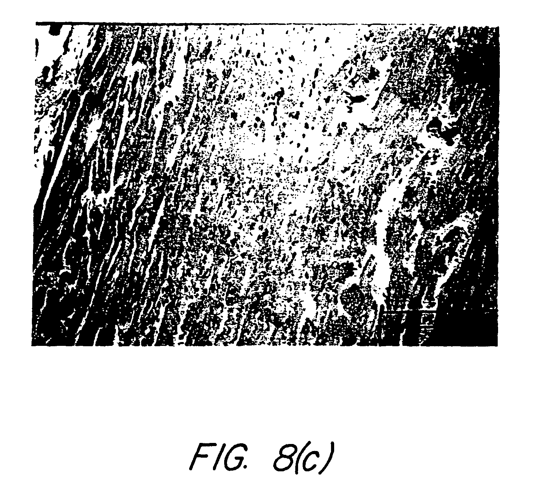

Discussion and conclusions of the results of the in vitro tests indicate that both Compound A and doxorubicin completely abolished [³H] thymidine incorporation into cells in each of the four cell lines studied. As shown by the ratio of IC₅₀ values (potency ratio), doxorubicin was more potent than Compound A to inhibit [³H] thymidine incorporation in all four cell lines (P<0.05). See Figs. 3-6. These studies demonstrate that both Compound A and doxorubicin are potent inhibitors of proliferation of cancer cells in vitro. Figs. 8a-c represent photomicrographs that illustrate the effects on heart tissue of a compound according to the present invention as compared to known doxorubicin compound and a control sample.

In vivo studies showed that Compound A was effective in prolonging survival in a mouse leukemia model with less systemic toxicity than doxorubicin as shown below.

The Effects of Compound A on P388 Leukemia in mice are described below.

CDF1 male mice were innoculated ip with 10⁶ P388 murine leukemia cells on day zero. On days 1 through 9 the mice were treated ip with doxorubicin or Compound A. Body weights were measured daily and survival was recorded. In one such study, mice were dosed with doxorubicin or Compound A at 0.8 mg/kg/day. At day 22 there were 0/8 survivors in the vehicle group, 7/8 in the doxorubicin group, and 5/8 in the Compound A group. The values for doxorubicin and Compound A were significantly different from the values for the vehicle but not from each other.

In another study with the same murine leukemia model, doxorubicin was injected at 0.8 mg/kg/day and Compound A was injected at 1.6, 2.4, or 3.2 mg/kg/day.

**Table 3**

| Dose mg/kg/day | Body weight gain, grams, day 19 | Survivors | |
|---|---|---|---|
| | | Day 25 | Day 35 |
| Vehicle | | | |
| 0 | 8.69±1.17 | 2/10 | 1/10 |
| Doxorubicin | | | |
| 0.8 | 3.44±1.34* | 8/10* | 1/10 |
| Compound A | | | |
| 1.6 | 3.32±0.85* | 8/10* | 1/10 |
| 2.4 | 1.23±0.46*^{#} | 8/10* | 0/10 |
| 3.2 | -0.87±0.53*^{#} | 9/10* | 8/10*^{#} |

| | | | |
|---|---|---|---|
| The values above are mean values ±SE; *p>0.05 versus vehicle; | | | |
| ^{#} p<0.05 versus doxorubicin. | | | |

At day 19, Compound A at 1.6 mg/kg/day was just as effective as doxorubicin in suppressing weight gain that resulted from the growth of the leukemia plus the associated ascites. Both the 2.4 and the 3.2 mg/kg/day doses of Compound A were more effective than doxorubicin in suppressing weight gain. At day 25 all doses of Compound A were as effective as doxorubicin in maintaining survival. By day 32, only Compound A, 3.2 mg/kg/day, was effective in prolonging survival compared to the vehicle and doxorubicin. The dose of doxorubicin used in this study is the maximally effective dose in this model. Higher doses of doxorubicin actually decrease survival. Thus, although Compound A is less potent than doxorubicin, it is more effective at higher doses than doxorubicin for prolonging survival.

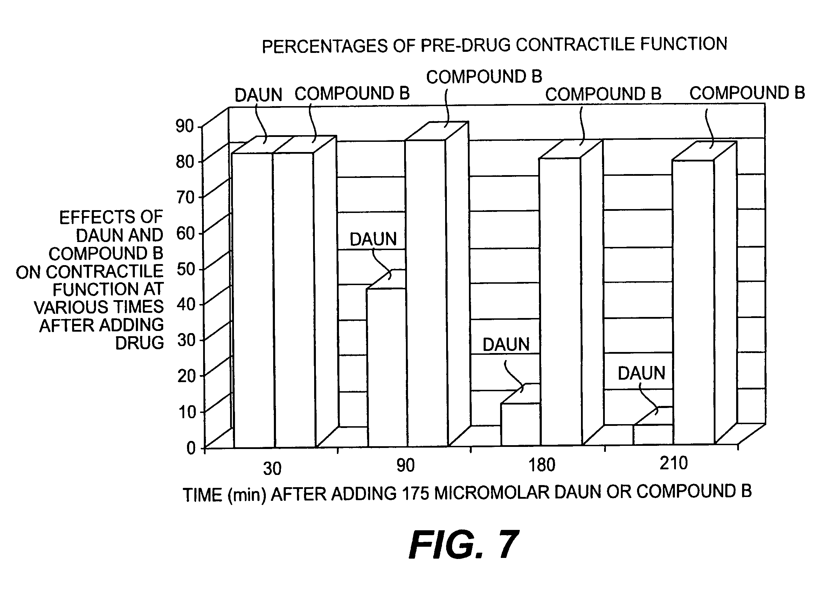

Compound B, the 13-deoxy analog of daunorubicin, has also been shown to be devoid of the cardiotoxic properties of daunorubicin in in vitro contractile heart function, using the rabbit heart model described in Mushlin et al., *supra*. This is illustrated in Fig. 7.

Compound B has also been shown to be devoid of the cardiotoxic properties of daunorubicin in an in vivo rat model, described below. The discussion below demonstrates the lack of cardiotoxic effects of Compound B according to the present invention in the rat following intravenous administration.

Daunorubicin hydrochloride or Compound B hydrochloride in water was injected intravenously at 5 mg/kg/day every other day for 3 days (total dose 15 mg/kg) in male Sprague Dawley rats. A separate vehicle group was studied with each compound. On day seven after the first dose each rat was anesthetized with sodium pentobarbital, 50 mg/kg ip. The trachea was intubated and the rat breathed 100% oxygen. Body temperature was maintained at 37°C with a heat lamp and a temperature controller. A catheter was placed in the right carotid artery and advanced into the aorta to record mean arterial pressure (MAP) and heart rate (HR) using a Statham pressure transducer and Gould recorder. The catheter was then advanced into the left ventricle to record left ventricular systolic pressure (LVSP), maximum left ventricular dP/dt (dP/dt), and left ventricular end diastolic pressure (LVEDP).

In rats treated with daunorubicin, MAP, LVSP, and dP/dt were significantly and substantially depressed, compared to vehicle controls (Table 4). Body weight was also significantly decreased in daunorubicin treated rats. In contrast, MAP, LVSP, dP/dt, and body weight were similar between vehicle and Compound B treated rats (Table 5). The data show that Compound B lacks cardiotoxicity at a dose at which daunorubicin produces substantial decreases in cardiac contractility and performance. The results illustrate that Compound B can be administered at a therapeutic dose without producing cardiotoxicity whereas this same therapeutic dose of daunorubicin produces impaired cardiac function.

**TABLE 4**

| Effects of daunorubicin and vehicle on left ventricular function in the rat after repeated dosing | | | | | | | |
|---|---|---|---|---|---|---|---|
| Treatment | MAP mmHg | HR LVSP b/minmmHg | | dP/dt mmHg/sec | LVEDP mmHg | BW1 gms | BW2 gms |
| Vehicle n=5 | 113 ±9 | 353 ±21 | 126 ±7 | 5,850 ±400 | 3.9 ±0.8 | 358 ±13 | 381 ±13 |
| Dauno-rubicin n=4 | 54* ±10 | 325 ±6 | 71* ±13 | 3,000* ±500 | 5.6 ±1.3 | 397 ±12 | 309* ±3 |

The values in Table 4 are means ± standard errors; rats were injected with compound at 5 mg/kg/day intravenously every other day for 3 days; measurements were made on day 7 after the first injection. BW1 = body weight on day zero, BW2 = body weight on day 7. * = p < 0.05 versus vehicle.

**TABLE 5**

| Effects of Compound B and vehicle on left ventricular function in the rat after repeated dosing | | | | | | | | |
|---|---|---|---|---|---|---|---|---|
| Treatment | Rat # | MAP mmHg | HR LVSP b/minmmHg | | dP/dt mmHg/sec | LVEDP mmHg | BW1 gms | BW2 gms |
| Vehicle | 1 | 125 | 350 | 145 | 5,500 | 2.81 | 380 | 389 |
| | 2 | 127 | 335 | 150 | 5,250 | 7.50 | 378 | 372 |
| Mean ±SE | | 126 ±1 | 343 ±8 | 148 ±3 | 5,375 ±125 | 5.20 ±2 | 379 ±1 | 381 ±9 |
| Compound B | 3 | 112 | 410 | 135 | 5,000 | 3.13 | 388 | 383 |
| | 4 | 125 | 340 | 163 | 6,850 | 7.50 | 373 | 373 |
| Mean ±SE | | 119 ±7 | 375 ±35 | 149 ±14 | 6,175 ±675 | 5.30 ±2.19 | 381 ±8 | 378 ±5 |

The rats were injected with compound at 5 mg/kg/day intravenously every other day for 3 days; measurements were made on day 7 after the first injection; BW1 = body weight on day zero, BW2 = body weight on day 7.

Compound A was also evaluated in a chronic doxorubicin cardiotoxicity model in the rabbit. In this model doxorubicin produces impaired cardiac function and histopathologic changes similar to that seen in humans treated chronically with doxorubicin. Histopathologic and/or functional impairment of the rabbit heart was observed in 5/6 rabbits treated with doxorubicin. Under the same conditions, Compound A produced no clinically relevant cardiotoxicity.

The non-cardiotoxic nature of the compound prepared according to the method of the present invention is supported the following study, which assesses cardiotoxicity of doxorubicin and Compound A in a chronic rabbit model.

According to the study, twenty four male New Zealand white rabbits were randomized into four groups. Six rabbits were injected with 1 mg/kg of doxorubicin into the marginal ear vein 2 times/week for 8 weeks. Six additional rabbits were injected with 1 mg/kg of Compound A into the marginal ear vein 2 times/week for 8 weeks. Food consumption of rabbits in the doxorubicin-treated and Compound A treated groups were monitored daily and the same amount of food was fed to sex and age-matched pair-fed control rabbits injected 2 times/week for 8 weeks into the marginal ear vein with the vehicle (0.9% NaCl). Aortic root acceleration was monitored weekly by doppler ultrasound technique for the duration of the study. Fractional shortening was determined every other week by M-mode echocardiography beginning the tenth week of the study and continuing for the duration of the study. Rabbits were euthanized beginning 20 weeks after the start of the study or when fractional shortening became smaller than 25% or remained 25-29% for at least 3 weeks. Left ventricular papillary muscle, left ventricular free wall and apex samples from each rabbit at sacrifice were prepared for histological analysis and graded by a histopathologist blinded to treatment. Lesions were graded as mild, moderate or severe based on degree of vacuolization, myofibrillar degeneration, mononuclear inflammation and necrosis. Abnormal fractional shortening occurred in 4/6, 0/6, 1/6 and 0/6 rabbits in the doxorubicin-treated, Compound A, doxorubicin control and Compound A control groups, respectively. Abnormal aortic root acceleration (values lower than 9 m/s/s) occurred in 3/6, 0/6, 0/6 and 0/6 rabbits in the doxorubicin-treated, Compound A, doxorubicin control and Compound A control groups, respectively. All 6 rabbits in the doxorubicin-treated group had abnormal histopathology ranging from mild to severe; 2/6 rabbits in the Compound A had mild histological abnormalities. No histopathological lesions were observed in cardiac tissue from both groups of control rabbits. The overall cardiac status was defined as abnormal when at least 2 of the 3 tests of cardiotoxicity were abnormal. Using these criteria, 5/6 rabbits in the doxorubicin-treated group had an overall cardiac status of abnormal; 0/6 were abnormal in the other 3 groups (P<0.05, Fisher's Exact test). Compared to doxorubicin, Compound A is essentially devoid of cardiotoxicity at the dose level tested. In addition, Compound A had no appreciable effect on hematology and body weight gain, whereas doxorubicin significantly altered hematology and depressed weight gain. At the present dosing schedule, Compound A produces less cardiotoxicity and systemic toxicity in rabbits compared to doxorubicin.

The purpose of the assessment of cardiotoxicity experiments included comparing the cardiotoxicity of Compound A with doxorubicin in a chronic rabbit model.

67% (4/6) of rabbits treated with doxorubicin developed abnormal left ventricular fractional shortening. Three of six doxorubicin-treated rabbits (50%) developed abnormal aortic root acceleration. In contrast, none of the rabbits treated with Compound A had any functional evidence of cardiotoxicity. The most sensitive indicator of cardiotoxicity was histopathology. All doxorubicin-treated rabbits elicited histopathological lesions characterized primarily by myocyte vacuolization and myofibrillar loss. Four of six rabbits exhibited mild cardiotoxicity, 1 rabbit had moderate lesions and 1 rabbit had severe lesions. Two of six Compound A treated rabbits had mild histopathological lesions (see Figure 8). When the results of all three cardiotoxicity tests were pooled, abnormal cardiac status was observed in 5 of 6 rabbits in the doxorubicin-treated group but 0 of 6 rabbits in the Compound A treated group (P<0.02, Fisher's Exact test). Overall cardiac status was defined as abnormal when at least 2 of the 3 tests of cardiotoxicity were abnormal). During the eighth week of the study, blood samples were collected from the marginal ear artery to obtain cell blood counts. Doxorubicin-treatment produced significant reductions in white cells, red cells, platelets, hemoglobin, mean corpuscular hemoglobin concentration, and red cell distribution width, compared to vehicle or Compound A treated animals, P>0.05. Compound A did not alter these variables compared to vehicle except for a slight increase in red cell distribution width. In addition, doxorubicin treatment inhibited weight gain compared to Compound A treatment. Compound A treated rabbits weighed 3.17±0.06 kg at the beginning of the study and 4.10±0.10 kg at the end of the study, whereas doxorubicin treated rabbits weighed 3.19±0.10 kg at the beginning of the study and 3.54±0.06 kg at the end of the study (P>0.05, 1 way anova, Duncan's New Multiple Range test).

Abnormal aortic root acceleration is defined as values below 9.0. Units of acceleration are m/s/s. N=normal cardiac function; A=abnormal cardiac function.

Rabbits were injected iv with 1 mg/kg doxorubicin (DOX) or Compound A (DOXA) 2 times/week for 8 weeks (total cumulative dose of 16 mg/kg). Age-matched, pair-fed controls for doxorubicin group (C) or Compound A group (CX) were injected with the vehicle only.

Doxorubicin group was significantly different than Compound A, CX or C group (P<0.05; 2X2 continguency Chi square analysis, two tail).

Rabbits were injected iv with 1 mg/kg doxorubicin (DOX) or Compound A (DOXA) 2 times/week for 8 weeks (total cumulative dose of 16 mg/kg). Age-matched, pair-fed controls for doxorubicin group (C) or Compound A group (CX) were injected with the vehicle only.

N=normal cardiac function or histopathology; A=abnormal cardiac function or histopathology. Overall cardiac status was defined as abnormal when at least 2 of the 3 tests of cardiotoxicity were abnormal. Abnonnal fractional shortening was defined as trends or sustained values in the mid-twenty percentiles or lower. Abnormal aortic acceleration was defined as values below 9.0 mists. Abnormal histopathology was defined as occurrence of vacuoles, myofibrillar lesions and mononuclear inflammation (see the above discussion of methods). Histopathology was scored as normal, mild, moderate or severe as previously described. Doxorubicin treated group had significantly more animals with abnormal overall cardiac status than Compound A, C and CX group (P<0.02; Fisher's Exact Test, two-tailed).

**Table 6**

| Cardiotoxic Evaluation of Compound A in Rabbits | | |
|---|---|---|
| | Incidence | |
| Cardiotoxic Endpoint | Doxorubicin₁ | Compound A |
| Depressed Fractional Shortening | 4/6 | 0/6 |
| Depressed Aortic Root Acceleration | 3/6 | 0/6 |
| Abnormal Histopathology₂ | 6/6 | 2/6 |
| Overall Cardiotoxicity | 5/6 | 0/6₃ |

| | | |
|---|---|---|
| ¹Dose: 1 mg/kg, twice per week, for 8 weeks | | |
| ²Abnormal Histopathology: vacuoles, myofibrillar lesions | | |
| ³p<0.02 versus doxorubicin. | | |

Among the conclusions of the cardiotoxicity studies were that Compound A did not alter cardiac function in the present and showed only mild histopathologic effects in 2/6 rabbits. On the other hand, doxorubicin altered cardiac function in 5/6 rabbits and all rabbits showed abnormal histopathology in this chronic rabbit model of cardiotoxicity. Compared to doxorubicin, Compound A is essentially devoid of cardiotoxicity at the dose level tested. In addition, Compound A had no appreciable effect on hematology and body weight gain. At the present dosing schedule, Compound A produces less cardiotoxicity and systemic toxicity in rabbits compared to doxorubicin.

In subacute toxicity tests in mice Compound A was also shown to produce less bone marrow toxicity than doxorubicin, as demonstrated below.

**Table 7**

| Effects of Compound A on red blood cells and bone marrow Lymphocytes in mice (n=4-5). | | | |
|---|---|---|---|
| Variable | Vehicle | Doxorubicin 12 mg/kg | Compound A 15 mg/kg |
| Erythrocytes, 10⁶/mm³ | | | |
| Male | 9.45±0.31 | 6.79±0.63+,# | 8.43±0.39 |
| Female | 9.22±0.19 | 6.51±0.35+,# | 8.59±0.41 |

| Hematocrit, % | | | |
|---|---|---|---|
| Male | 45.6±1.90 | 32.2±2.80+,# | 41.1±1.48 |
| Female | 46.1±1.01 | 31.1±1.58+,# | 43.5±2.32 |

| Bone marrow lymphocytes Percentage of total | | | |
|---|---|---|---|
| Male | 22.2±2.6 | 6.3±0.36+ | 8.6±2.41+ |
| Female | 35.3±6.00 | 8.5±0.50+ | 10.5±2.06+ |
| The drugs were administered intravenously on days 1, 5, and 9. Measurements were made on day 15. Values are means ±SE. +=different from vehicle, p<0.05, #=different from Compound A, p<0.05. Both doses are maximum sublethal doses. | | | |

The results of the above-described studies clearly demonstrate that Compound A is a noncardiotoxic form of doxorubicin. Because Compound A retains the 14-OH moiety it is probable that Compound A will be useful in sarcomas and carcinomas in addition to leukemias. No dose limiting cardiotoxicity is expected to occur because Compound A does not form a toxic 13-alcohol metabolite. Consequently, Compound A, unlike doxorubicin, may be administered as long as necessary to produce remission and/or to prevent recurrence and metastases. In this regard, Compound A and other 13-deoxyanthracyclines represent a major breakthrough in anthracycline chemotherapy of cancer.

The results demonstrate that anthracycline derivatives like Compound A should be more effective clinically than their non-13-deoxyanthracycline counterparts since they may be given at higher efficacious doses and for longer periods of time because they produce less systemic toxicity and no dose limiting cumulative cardiotoxicity. The 13-deoxyanthracycline derivatives employed according to the present invention in treating patients suffering from cancers treatable with doxorubicin and daunorubicin, exhibit the capability of being administered in dosages of at least about 1.5 times the effective or equipotent cumulative dosage compared to the 13-keto counterpart compounds.

Table 8 provides examples of 13-deoxyanthracycline derivatives that are synthesized according to the method of the present invention. As discussed above, compounds such as those shown in Table 8 are known to have anti-tumor properties.

Unlike known processes, the processes of the present invention are less temperature sensitive. For example, the processes may be carried out at a temperature of from about 0°C to about 75°C. Preferably, the processes are carried out at a temperature of from about 65°C to about 75°C. More preferably, the process are carried out at a temperature of from about 68°C to about 72°C. Temperatures over about 72°C typically result in decomposition of the reactants and products.

The process of the present invention includes a number of general conditions. For example, the processes preferably are carried out in acidic conditions. In other words, the pH should be about 6.5 or less. Known processes for preparing the above compounds, which employ basic conditions within the reaction mixture, have been found to cause decomposition of the reactants and products. The reaction, or any part thereof, such as only the refluxing, may be carried out at a temperature of up to about 75°C, in an absence of oxygen, in an absence of water, and/or under nitrogen.

Additionally, both oxygen and water should be excluded from the reactions. Preferably, the reaction is conducted in a nitrogen or inert gas atmosphere, using anhydrous solvents.

The processes of the present invention result in a much higher yield than known processes for preparing the compounds. For example, known processes have been found to have a yield of about 30%. On the other hand, processes of the present invention have been found to have a yield of from about 70% to about 80%.

In accordance with the above, the present invention provides processes for preparing compounds of the general formula I above.

The following provides an example of the transformation of the molecule as it progresses through the process. 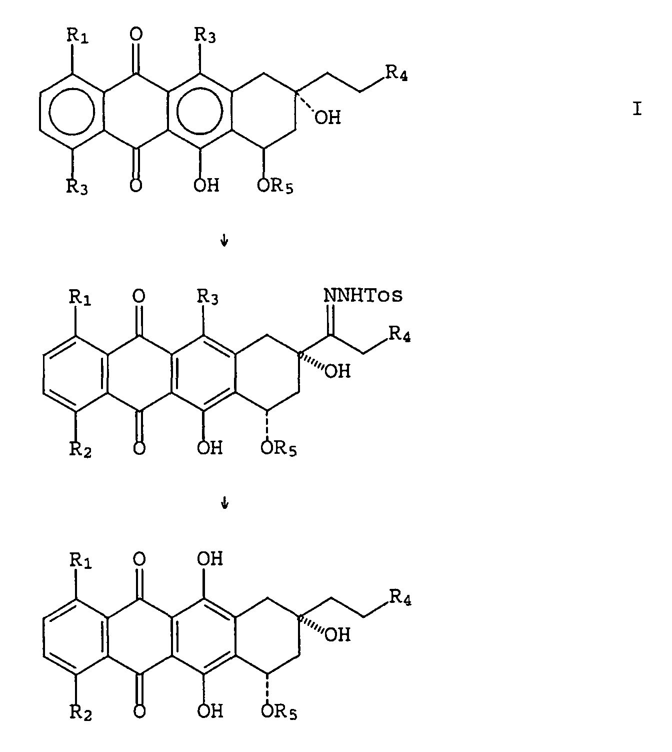 wherein R₁, R₂, R₃, R₄, and R₅ are as defined above.

The following flowchart illustrates an example of an embodiment of a method according to the present invention for producing 13-deoxydoxorubicin, which is a 13-deoxyanthracycline derivative.

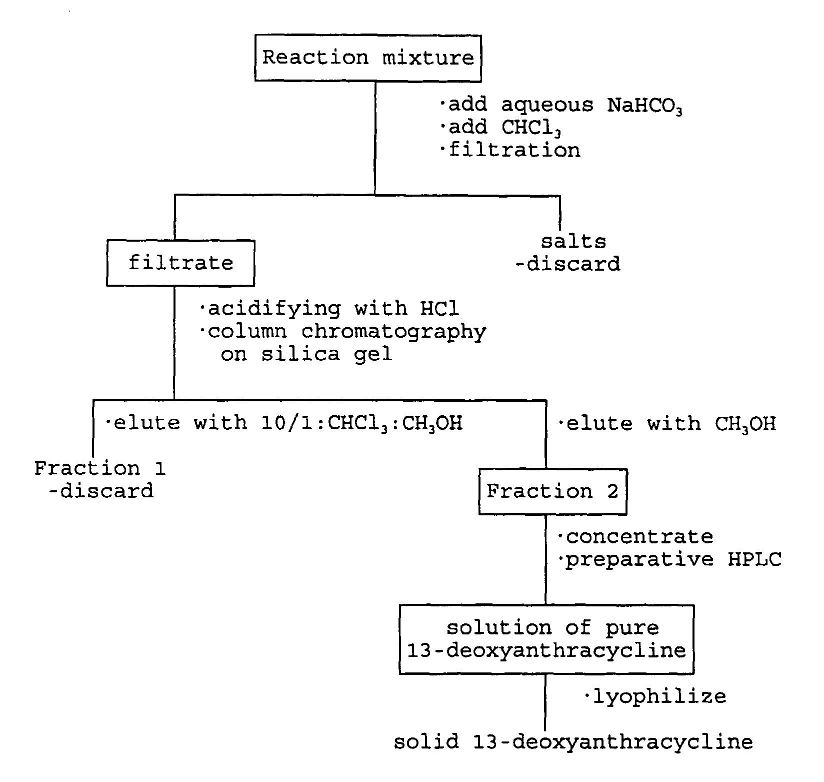

The following represents examples of anthracycline derivatives, the synthesis of which is disclosed herein.

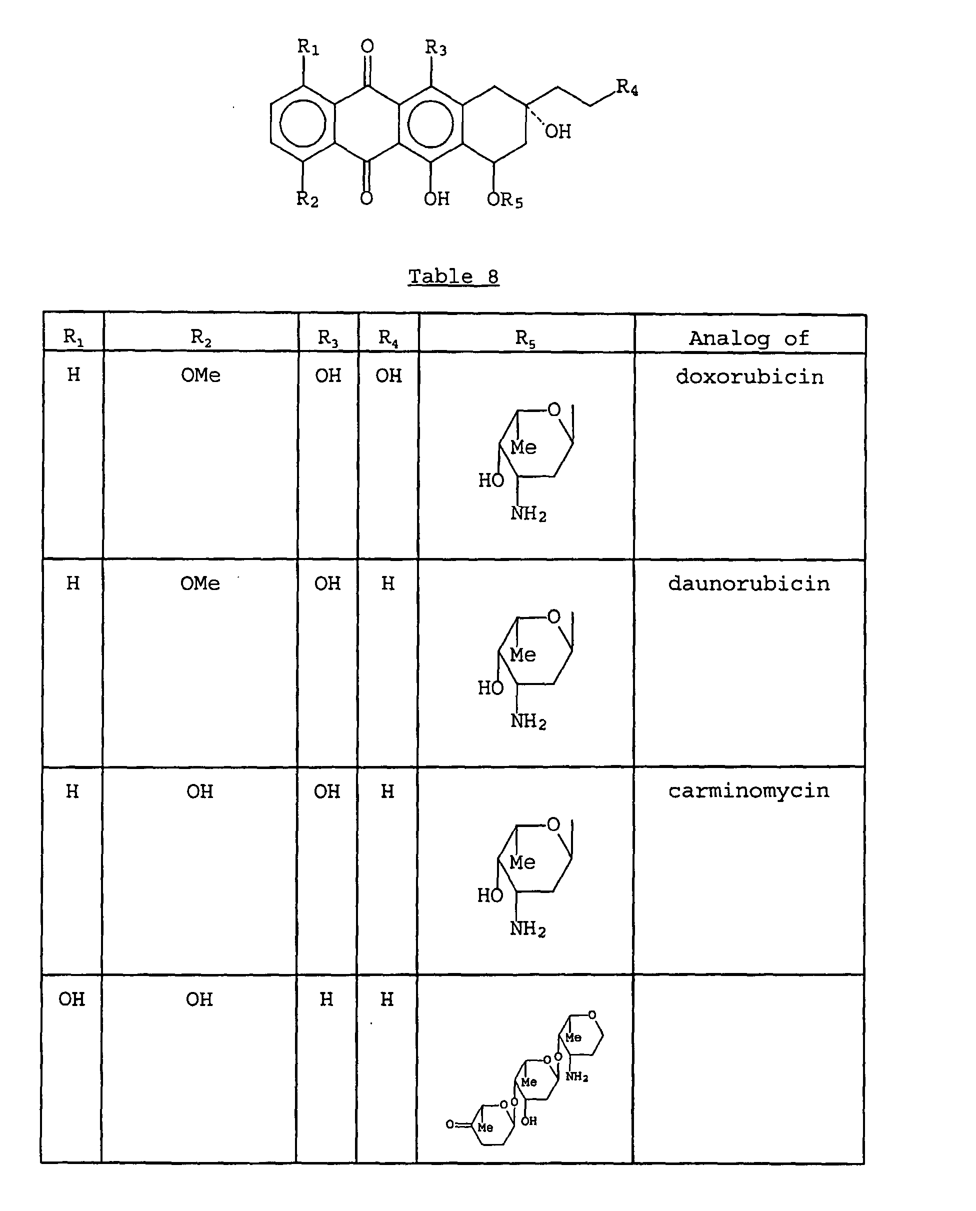

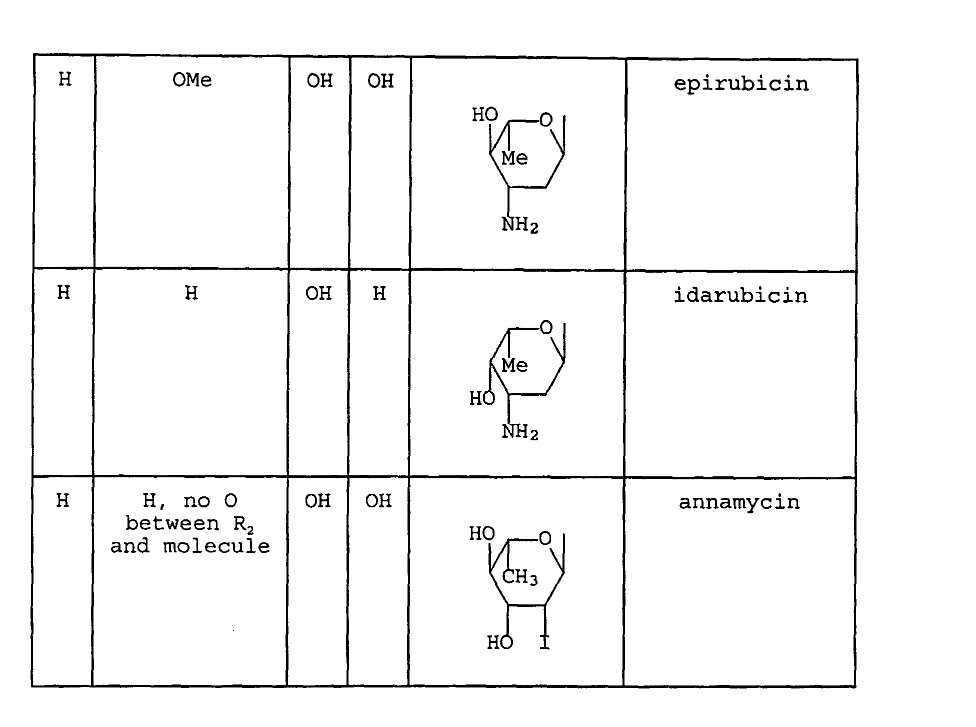

In the compounds, R₅ may be a modified version of different anthracycline analogs. Also, The D ring may be fluorinated.

Generally, processes according to the present invention include forming a solution of a 13-deoxyanthracycline with a reducing agent. The solution is gently refluxed. Then, the reaction mixture may be cooled. According to one example, the reaction mixture is cooled to a temperature of from about 0°C to about 4°C. A base is then added to the reaction mixture. The base may be cold. For example, the base be at a temperature of from about 0°C to about 4°C. One example of a base is saturated aqueous NaHCO₃. A halocarbon solvent may be added to the reaction mixture. The halocarbon solvent may be added to the reaction mixture simultaneously with the base. The halocarbon solvent may be cold. For example, the halocarbon solvent may be at a temperature of from about 0°C to about 4°C. An example of a halocarbon solvent that may be utilized is CHCl₃. The reaction mixture may then be filtered. The filtration may also take place at a reduced temperature. For example, the filtration may take place at a temperature of from about 4°C to about 15°C.

Addition of the base and the halocarbon solvent described above preferably initiates a hydrolysis precipitation. It is the precipitate of inorganic salts that may be filtered out of the reaction mixture. After filtration, the filtrate may be acidified. The filtrate may be subjected to column chromatography on silica gel. Hydrophobic impurities may be isolated by eluting with less polar solvents. 13-deoxyanthracycline products may then be eluted and the elute further purified.

Preferably, the processes according to the present invention include forming a solution of anthracycline 13-tosylhydrazones in anhydrous methanol with p-toluenesulfonic acid and sodium cyanoborohydride. The solution is refluxed gently under nitrogen and then cooled. Saturated aqueous sodium bicarbonate and chloroform are added. Salts precipitated are filtered and the filtrate is acidified with hydrogen chloride in diethyl ether and then isolated on a silica gel column. The hydrophobic impurities resulted from decomposition are eluted with chloroform and methanol mixed solution. The products, 13-deoxyanthracyclines, are eluted with methanol. The methanol elute is further purified by preparative HPLC.

According to any of the processes described above, prior to or after isolation of the 13-deoxyanthracyclines, the 13-deoxyanthracyclines may be treated with one or more reducing and/or other agents capable of reducing the 13-keto moiety to a methylene moiety.

The following provides an example of a process according to the present invention.

### Example

### Preparation of 13-Deoxydoxorubicin hydrochloride

1 g of doxorubicin 13-tosylhydrazone hydrochloride and 2.4 g of p-toluenesulfonic acid are dissolved in 50 mL of anhydrous methanol. To this solution 0.8 g of sodium cyanoborohydride is added. The resulting solution is heated to 68-72°C and kept at gentle reflux for one hour under a nitrogen atmosphere.

Then, the reaction mixture is concentrated to about 20 mL and cooled in a freezer to 0-4°C. 2 mL of saturated aqueous sodium bicarbonate is added followed by 200 mL of chloroform. Anhydrous sodium sulfate is added and the salts are filtered after shaking. The filtrate is acidified with hydrogen chloride in diethyl ether.

The solution is then run through a silica gel column (2.5 x 5 cm). The column is further washed with chloroform/methanol (10/1) until the eluate is colorless. The bound fraction containing the product is eluted with methanol. The methanol eluate is evaporated and residue is dissolved in 30% acetonitrile in ammonium formate buffer (pH = 4.0, 0.5%) and isolated by preparative HPLC. A phenyl column is used and separation of the product from the other impurities is achieved by using an acetonitrile/ammonium formate gradient (from 27% to 30% acetonitrile for 30 min). The HPLC purified fraction is lyophilized to give solid 13-deoxydoxorubicin hydroformate, which is then dissolved in methanal containing hydrogen chloride. The solvent is evaporated and the produce is precipitated in methanol/ethyl ether to give 600 mg 13-deoxydoxorubicin hydrochloride. The yield is 80%.

| | | | | |
|---|---|---|---|---|
| TLC | R_{*f*}=0.38 | CHCl₃ | MeOH | H₂O |
| | | 30 | 10 | 1 |

U.V.: λₘₐₓ=233, 252, 293, 485 nm
MS: 530(M+H), 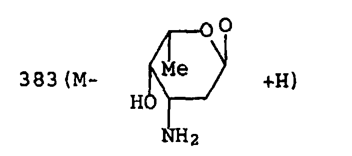 ¹HNMR (methanol d₄): (see below)
δ 1.30 (d, 3H, 6'-H₃),
1.85 (m, 2H, 13-H₂),
2.05 (m, 2H, 10-H₂),
2.60 (d, 1H, 12-H),
3.05 (d, 1H, 12-H),
3.55 (m, 1H, 5'-H),
3.90 (m, 2H, 14-H₂),
4.05 (m, 3H, O-CH₃),
4.25 (m, 1H, 4'-H),
4.95 (m, 1H, 3'-H),
5.40 (m, 1H, 1'-H),
7.50 (dd, 1H, 3-H), and
7.80 (m, 2H, 1-and 2-H).

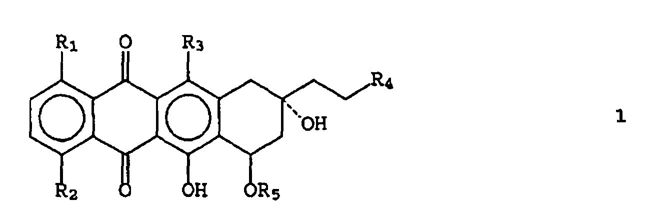

The effective anticancer amount of the compound prepared according to the method of the present invention may be administered dependent upon the species of the mammal, the body weight, age, and individual condition, as well as upon the form of administration. The compounds of the present invention can be administered by conventional means available for use in conjunction with pharmaceuticals, either as individual therapeutic agents or in a combination of therapeutic agents. They can be administered alone, but generally administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms, the kind of concurrent treatment; the frequency of treatment; and the effect desired. A daily dosage of active ingredient can be expected to be about 0.001 to 1000 milligram (mg) per kilogram (kg) of body weight, with the preferred dose being 0.1 to about 30 mg/kg.

Dosage forms (compositions suitable for administration) contain from about 1 mg to about 100 mg of active ingredient per unit. In these pharmaceutical compositions, the active ingredient will ordinarily be present in an amount of about 0.5-95% by weight based on the total weight of the composition.

The active ingredient can be administered orally in solid dosage forms, such as capsules, tablets, and powders, or in liquid dosage forms, such as elixirs, syrups, and suspensions. It can also be administered parenterally, in sterile liquid dosage forms. The active ingredient can also be administered intranasally (nose drops) or by inhalation. Other dosage forms are potentially possible such as administration transdermally, via a patch mechanism or ointment.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water-soluble salt of the active ingredient, suitable stabilizing agents, and, if necessary, buffer substances. Antioxidizing agents such as sodium bisulfite, sodium sulfite, or ascorbic acid, either alone or combined, are suitable stabilizing agents. Also used are citric acid and its salts and sodium EDTA.

In addition, dosage forms for intravenous or i.p. administration can contain lyophilized powder for reconstitution with sterile water or sterile saline for injection. These solutions can contain preservatives, such as benzalkonium chloride, methyl- or propylparaben, and chlorobutanol.

Suitable pharmaceutical carriers are described in Remington's Pharmaceutical Sciences, Mack Publishing Company, a standard reference text in this field.

## Claims

1. A process for preparing 13-deoxyanthracycline derivatives, said process comprising the steps of:
forming a solution of anthracycline 13-tosylhydrazone;
adding a reducing agent to reduce the 13-keto moiety to a methylene moiety;
refluxing the solution;
cooling the reaction mixture;
adding saturated aqueous NaHCO₃;
adding a halocarbon solvent to the reaction mixture;
filtering the reaction mixture;
acidifying the filtrate; and
subjecting the filtrate to chromatography to isolate the 13-deoxyanthracycline derivatives.

2. A process for the preparation of 13-deoxyanthracycline derivatives, said process comprising the steps of:
forming a solution of anthracycline 13-tosylhydrazone in anhydrous methanol with p-toluenesulfonic acid and sodium cyanoborohydride;
gently refluxing the solution under nitrogen;
cooling the solution;
adding saturated aqueous sodium bicarbonate and chloroform to the solution to form a precipitate;
filtering the precipitate;
acidifying the filtrate with hydrogen chloride in diethyl ether;
isolating salt contained in the filtrate on a silica gel column;
eluting the hydrophobic impurities resulting from decomposition of the salts with a mixed solution of chloroform and methanol;
eluting the 13-deoxyanthracycline products with methanol; and further purifying the methanol elute by preparative HPLC.

3. The process according to claim 2, wherein said solution has a pH of 6.5 or less.

4. The process according to claim 2, wherein said refluxing is carried out at a temperature of from about 68°C to about 72°C.

5. The process according to claim 2, wherein said refluxing is carried out at a temperature of from about 65°C to about 75°C.

6. The process according to claim 2, wherein said refluxing is carried out at a temperature of up to about 75°C.

7. The process according to claim 2, wherein said refluxing is carried out in an absence of oxygen.

8. The process according to claim 2, wherein said refluxing is carried out in an absence of water.

9. The process according to claim 2, wherein said refluxing is carried out in an atmosphere of nitrogen.

10. The process according to claim 2, wherein said refluxing is carried out in an atmosphere of an inert gas.

11. The process according to claim 2, wherein said process results in a yield of from about 70% to about 80%.

12. A process for the preparation of 13-deoxyanthracycline derivatives, said process comprising the steps of:
forming a solution of anthracycline 13-tosylhydrazone;
adding a reducing agent;
refluxing the solution;
cooling the reaction mixture;
adding saturated aqueous NaHCO₃;
adding a halocarbon solvent to the reaction mixture;
filtering the reaction mixture;
acidifying the filtrate; and
subjecting the filtrate to chromatography to isolate the 13-deoxyanthracycline derivatives.

13. A process for the preparation of 13-deoxyanthracycline derivatives, said process comprising the steps of:
forming a solution by dissolving about 1 g of doxorubicin 13-tosylhydrazone hydrochloride and about 2.4 g of p-toluene sulfonic acid in about 50 mL of anhydrous methanol;
adding about 0.8g of sodium cyanoborohydride 60 the solution;
heating the solution to a temperature of from about 68°C to about 72°C;
gently refluxing the solution for about one hour under a nitrogen atmosphere;
concentrating the reaction mixture to about 20 mL;
cooling the reaction mixture in a freezer to a temperature of from about 0°C to about 40°C;
adding about 2 mL of saturated aqueous sodium bicarbonate to the reaction mixture;
adding about 200 mL of chloroform to the reaction mixture;
adding anhydrous sodium sulfate;
filtering salts resulting from the addition of the anhydrous sodium sulfate after shaking;
acidifying the filtrate with hydrogen chloride in diethyl ether;
running the solution through a silica gel column;
further washing the column with chloroform/methanol until the eluate is colorless;
eluting with methanol a fraction containing the product;
evaporating the methanol eluate;
dissolving residue resulting from the evaporation in 30% acetonitrile in ammonium formate buffer;
isolating the produce by preparative HPLC using a phenyl column;
separating the product from other impurities using an acetonitrile/ammonium formate gradient; and
lyophilizing the HPLC purified fraction to produce about 600 mg of 13-deoxydoxorubicin hydrochloride.
